Europäisches Patentamt

European Patent Office

Office européen des brevets

(19)

(11) Numéro de publication: **0 145 607**
**B1**

(12) **FASCICULE DE BREVET EUROPÉEN**

(45) Date de publication du fascicule du brevet:
27.04.88

(51) Int. Cl.⁴: **A 61 K 7/48**

(21) Numéro de dépôt: **84402564.3**

(22) Date de dépôt: **12.12.84**

(54) Composition cosmetique aqueuse ou anhydre contenant une phase grasse a base d'huile de karite.

(30) Priorité: **12.12.83 LU 85130**

(43) Date de publication de la demande:
**19.06.85 Bulletin 85/25**

(45) Mention de la délivrance du brevet:
**27.04.88 Bulletin 88/17**

(84) Etats contractants désignés:
**AT BE CH DE FR GB IT LI NL**

(56) Documents cité:
**FR-A-1 269 621**
**GB-A-2 102 290**

(73) Titulaire: **L'OREAL, 14, Rue Royale, F-75008 Paris
(FR)**

(72) Inventeur: **Zabotto, née Arribau, Arlette, 24 rue
Sarrette, 75014 Paris (FR)**
Inventeur: **Griat, née Le Calvez, Jacqueline, 11,
Quai de la Baronnie, 94480 Ablon (FR)**
Inventeur: **Bracco, Umberto, Entre- deux- crêts 20,
CH- 1814 La Tour de Peilz (CH)**

(74) Mandataire: **Nony, Michel, Cabinet Nony 29, rue
Cambacérès, F-75008 Paris (FR)**

# 0 145 607

## Description

La présente invention a pour objet de nouvelles compositions cosmétiques, aqueuses ou anhydres, dont la phase grasse est essentiellement à base d'une huile végétale ci-après désignée sous le terme d'"huile de karité", éventuellement en mélange avec d'autres huiles, graisses ou cires.

La plupart des compositions cosmétiques contiennent à une concentration plus ou moins élevée, une phase grasse généralement constituée d'un mélange d'au moins une huile, une graisse et/ou une cire. Il en est tout particulièrement ainsi des émulsions du type huile-dans-l'eau ou eau-dans-l'huile, des gels, des huiles pour les soins du corps et du visage, des laits et des produits de maquillage tels que des fards ou des bâtons pour les lèvres.

Les huiles constituent une proportion importante de ces phases grasses et peuvent être choisies dans une large gamme de produits qu'ils soient d'origine naturelle ou synthétique.

Du fait de leurs propriétés, l'on préfère généralement utiliser des huiles naturelles, notamment des huiles végétales, mais se pose alors le problème de la constance de leurs caractéristiques en fonction de leur provenance, des traitements subis, et de leur coût généralement élevé.

Ainsi, l'huile de jojoba, dont les excellentes propriétés cosmétiques ont été amplement reconnues par différents auteurs et qui est largement utilisée dans de nombreuses compositions cosmétiques (voir en particulier GB-A-2.102.290), est une huile relativement chère sur le marché mondial et ne présente pas toujours en fonction de sa provenance et de son procédé d'extraction, des caractéristiques physiques constantes ce qui non seulement influe sur le prix de revient mais également sur une bonne reproductibilité des compositions la contenant.

On vient maintenant de constater, après de nombreuses recherches, qu'il était possible d'obtenir d'excellentes compositions cosmétiques, contenant une phase grasse, en utilisant de l'huile de karité ou oléine de karité, obtenue à partir du beurre de karité, cette huile n'ayant jamais été, à la connaissance de la Société déposante, encore préconisée pour constituer un corps gras d'une phase grasse d'une composition cosmétique.

Cette huile, bien que différente de par ses caractéristiques de l'huile de jojoba, confère aux compositions cosmétiques des propriétés très similaires, notamment par son effet protecteur et assouplissant de la peau.

On a également constaté que l'association de cette huile avec au moins un acide gras essentiel ou leurs mélanges, notamment avec la vitamine F, présentait une bonne stabilité à l'oxydation.

Par ailleurs, l'huile de karité s'est montrée présenter un bon pouvoir protecteur des érythèmes provoqués par les ultra-violets (UVB)

La présente invention a pour objet à titre de produit industriel nouveau, une composition cosmétique, aqueuse ou anhydre, dont la phase grasse est essentiellement constituée par de l'huile de karité, ou par un mélange d'huile de karité et d'au moins une autre huile, une graisse et/ou une cire cosmétique, ladite huile de karité provenant de l'extraction, à l'aide d'un solvant organique, du beurre de karité.

Le beurre de karité provient lui-même des fruits (amandes) de l'arbre (Butyrospermum parkii) que l'on rencontre au Mali, au Soudan, au Sénégal et au Gabon. L'amande contient de 45 à 55 % de matière grasse que l'on extrait et que l'on raffine.

Le beurre de karité se présente sous forme d'un produit jaune ayant les caractéristiques suivantes:

| | |
|---|---|
| - Acide palmitique ($C_{16}$) | 5-7 %, |
| - Acide stéarique ($C_{18}$) | 40-44 %, |
| - Acide oléique ($C_{18:1}$) | 48-51 %, |
| - Acide linoléique ($C_{18:2}$) | 2-5 %, |
| - Acide arachidique ($C_{20}$) | 0-0,5 % |
| (ces pourcentages étant exprimés par rapport au poids total des acides gras) | |
| - poids spécifique à 15°C: | 0,915-0,918 |
| - Indice de réfraction à 40°C: | 1,4633-1,4668 |
| - Indice d'iode: | 51-60 |
| - Indice de saponification: | 170-185 |
| - Point de fusion: | 37-38°C. |

A partir du beurre de karité on peut obtenir deux fractions, l'une dite solide ou "stéarine du beurre de karité" connue comme substitut du beurre de cacao en chocolaterie et en confiserie, l'autre dite liquide ou "huile du beurre de karité".

La fraction liquide résulte du fractionnement en continu et/ou en discontinu du beurre de karité à l'aide d'un solvant organique tels que les alcanes, les cétones, les alcools ou leurs mélanges binaires et ternaires azéotropiques.

De préférence, l'extraction est réalisé à l'aide d'acétone à des températures comprises entre 12 et 20°C, ceci dépendant du rapport entre le beurre de karité et le solvant organique utilisé. Après filtration à froid, la friction solide ou stéarine est séparée et la phase solvant est évaporée conduisant à l'huile de karité qui représente environ 1/3 en poids du beurre de karité soumis à l'opération de fractionnement.

L'huile de karité ainsi obtenue présente les caractéristiques suivantes:

2

| | |
|---|---|
| - Acide palmitique ($C_{16}$) | 3-5 % |
| - Acide stéarique ($C_{18}$) | 22-30 % |
| - Acide oléique ($C_{18:1}$) | 55-65 % |
| - Acide linoléique ($C_{18:2}$) | 0,5-1,5 % |
| - Acide arachidique ($C_{20}$) | 0,5-1,5 % |

(Ces pourcentages étant exprimés par rapport au total des acides gras)

| | |
|---|---|
| - Viscosité: | 100-300 mPa.s (cP) |
| - Point de fusion: | 5,1-5,4°C |
| - Cristallisation: | 2,9-3°C |
| - Insaponifiable: | 1,2-1,5 % |
| - Composition de l'insaponificable (en % de l'insaponifiable total) | |
| - Alcanes: | 15-18 % |
| - Alcools à longue chaîne: | 10-12 % |
| - Stérols: | 60-66 % |
| - Alcools triterpéniques: | 10-15 % |

Les stérols de l'insaponifiable sont essentiellement les suivants:

| | |
|---|---|
| - Campestérol | 10 ± 2 % |
| - Stigmastérol | 5 ± 1 % |
| - β sitostérol | 52 ± 4 % |
| - Δ 5 Avenastérol | 15 ± 2 % |
| - Δ 7 Avenastérol | 10 ± 1 % |

(Ces pourcentages étant exprimés par rapport aux stérols totaux)

Les alcools triterpéniques de l'insaponifiable exprimés en % des alcools triterpéniques totaux sont les suivants:

| | |
|---|---|
| - Cycloartanol | 2 % |
| - α Amyrin | 46 ± 2 % |
| - β Amyrin | 10 ± 1 % |
| - Butyrospermol | 26 ± 1 % |
| - Cycloartenol | 2 % |
| - Lupéol | 16 + 1 % |
| - 24-méthyléne Cycloarténol: | traces. |

De préférence l'huile de karité loit présenter les rapports d'acides gras suivants:

$$\frac{C_{16} + C_{18}}{C_{18:1}} = 0,518 \pm 0,01$$

$$\frac{C_{16}}{C_{18:1}} = 0,064 \pm 0,003$$

**Exemple de préparation de l'huile de karité**

1000 g de beurre de karité sont dissous à 40°C dans 7.000 ml d'acétone et le mélange est ensuite refroidi à 15°C.

Après 6 heures, on filtre à froid pour obtenir la fraction solide que l'on sépare et la phase solvant qui contient la fraction liquide de karité. On évapore par distillation le solvant qui est recyclé et on obtient environ 300 g le fraction liquile d'huile de karité.

L'huile le karité ainsi obtenue présentait les caractéristiques suivantes:

3

| | |
|---|---|
| - Acide palmitique ($C_{16}$) | 3,8 ± 0,18 |
| - Acide stéarique ($C_{18}$) | 26,8 ± 0,12 |
| - Acide oléique ($C_{18:1}$) | 59,0 ± 0,23 |
| - Acide linoléique ($C_{18:2}$) | 0,9 ± 0,18 |
| - Acide arachidique ($C_{20}$) | 0,8 ± 0,1 |
| - Viscosité: | 170 cps |
| - Point de fusion: | 5,3° C |
| - Cristallisation: | 2,95° C |

Selon l'invention l'huile de karité est présente à une concentration d'environ 1 à 80 % en poids par rapport au poids total de la composition cosmétique, cette concentration étant de préférence de 2 à 30 % en poids dans le cas d'une émulsion et pouvant aller jusqu'à 80 % environ dans le cas d'un baume anhydre.

Les autres constituants de la phase grasse sont soit des huiles végétales ou animales soit des huiles minérales ou encore des huiles synthétiques.

Par ailleurs, la phase grasse peut également contenir, en une certaine proportion, des graisses et/ou des cires.

Parmi les huiles végétales ou animales, modifiées ou non, on peut citer par exemple l'huile d'amande douce, l'huile d'avocat, l'huile de ricin, l'huile d'olive, l'huile de jojoba, l'huile de sésame, l'huile de soja, l'huile de colza, le perhydrosqualène, l'huile de calophyllum, la lanoline et ses dérivés.

Parmi les huiles minérales on peut citer par exemple, l'huile de vaseline et parmi les huiles synthétiques, le palmitate d'éthyle et d'isopropyle, les myristates d'alkyle tel que le myristate d'isopropyle, de butyle, de cétyle, le stéarate d'hexyle, les triglycérides des acides octanoïques et décanoïques (par exemple le produit vendu sous la dénomination de "MIGLYOL 812 ®") par la Sociéte DYNAMIT NOBEL, le ricinoléate de cétyle, l'octanoate de stéaryle (huile de purcellin), le polyisobutène hydrogéné et les huiles de silicone solubles dans les autres huiles tels que par exemple le diméthyl polysiloxane ou le méthylphényl polysiloxane.

Parmi les cires on peut en particulier citer la cire de carnauba, la cire d'abeille, l'ozokérite, la cire de candellila, la cire de Montan et les circs microcristallines.

Ces huiles et ces cires sont d'une utilisation courante en cosmétique et sont bien connues des cosméticiens comme étant susceptibles de constituer des substances propres à la réalisation de phases grasses ou huiles de divers produits cosmétiques.

En plus des huiles et des cires ci-dessus mentionnées, la phase grasse selon l'invention peut également contenir certains composés considérés comme des produits gras, à savoir des alcools à longue chaîne tels que l'alcool cétylique, l'alcool stéarylique, l'alcool myristique, l'alcool hydroxystéarylique, l'alcool oléique ou l'alcool isostéarylique.

Ces derniers composés lorsqu'ils sont présents dans la phase grasse, représentent généralement de 0,5 à 5 % du poids total de la composition.

La phase grasse peut également contenir certains polymères tel que par exemple le polylaurate de vinyle.

D'une façon générale la phase grasse est présente en une proportion d'environ 4 à 99,9 % en poids du poids total des compositions cosmétiques.

Les compositions cosmétiques selon l'invention sont d'une façon générale toutes les compositions aqueuses ou anhydres contenant une phase grasse.

Parmi ces compositions, on peut en particulier citer celles qui se présentent sous forme d'émulsions fluides, de lotion ou sous forme d'émulsions plus consistantes.

Ces compositions sont par exemple des laits ou des crèmes émollients, des laits ou des crèmes pour les soins des mains, des crèmes ou des laits démaquillants, des bases de fond de teint, des laits ou des crèmes "anti-solaires", des laits ou des crèmes de bronzage artificiel, des laits ou des crèmes contre la transpiration, des crèmes ou des mousses de rasage.

Selon une première forme de réalisation, les compositions cosmétiques selon l'invention peuvent être essentiellement constituées par la phase grasse et se présentent alors sous forme d'une huile anti-solaire (contenant un filtre solaire absorbant l'ultra-violet), d'une huile pour les mains, d'une huile pour le corps ou les cheveux, d'une huile de pré-rasage ou d'après-rasage, d'une huile pour le bain, d'un gel, d'un baume, d'un stick.

Lorsque les compositions se présentent sous forme de crèmes ou de laits, il s'agit plus particulièrement d'émulsions du type eau-dans-l'huile ou huile-dans-l'eau dont la phase grasse représente de 4 à 60 % en poids, la phase eau de 30 à 90 % et l'agent émulsionnant de 1 à 20 %, de préférence de 2 à 12 % en poids.

Parmi les agents émulsionnants on peut en particulier citer:

- les alcools gras polyoxyéthylénés ou polyglycérolés, les sulfates d'alkyle oxyéthylénés ou non, les mélanges d'au moins un lanolate tels que les lanolates de magnésium, calcium, lithium, zinc ou aluminium et de lanoline hydrogéné et/ou d'alcool de lanoline, des esters d'acides gras et de polyols tels que le glycérol ou le propylène glycol.

- des monoesters d'acides gras et de sorbitan polyoxyéthylénés par exemple les produits vendus par la Société ATLAS sous la dénomination de "TWEEN®".

Ces compositions peuvent également contenir d'autres ingrédients conventionnels tels que des agents épaississants ou des gélifiants comme par exemple:

- des silicates de magnésium et d'aluminium,

4

- des copolymères éther-vinylique/anhydride maléique tels que les produits vendus sous la dénomination de "VISCOFAS X 100.000 "VISCOFAS L 100 ®" par la Société I.C.I. associés à des amino-acides.

- ou des polymères carboxyvinyliques tels que les produits vendus sous la dénomination "CARBOPOL R" par la Société GOODRICH.

- ou encore des gels de montmorillonite modifiée organiquement et d'huile neutre par exemple le produit "Miglyol gel®" vendu par la Société DYNAMIT NOBEL.

Les compositions selon l'invention contiennent en outre divers ingrédients, notamment des agents colorants, des parfums, des conservateurs, des filtres UV, des pigments, des agents nacrants et des charges minérales ou organiques.

Selon une forme de réalisation préférée de l'invention, l'huile de karité est associée à au moins un acide gras essentiel ou un mélange de ces acides, notamment à la vitamine F, sous forme acide libre ou estérifiée, l'association présentant une bonne stabilité à l'oxydation.

Par l'expression "acide gras essentiel" on doit entendre un acide gras insaturé possédant au moins deux doubles liaisons tel que:

1) l'acide linoléique ou acide 9,12 octadécadiénoïque et ses stéréoisomères et notamment l'isomère Z-9, Z-12 ainsi que ses isomères de position ou acides linoléiques conjugués à savoir: l'acide 9,11-octadécadiénoïque et ses stéréoisomères, et l'acide 10-12 octadécadiénoïque et ses stéréoisomères,

2) l'acide α linolénique ou acide 9,12,15 octadécatriénoïque et ses stéréoisomères et notamment l'isomère Z-9, Z-12, Z-15.

3) l'acide γ linolénique ou acide 6, 9,12-octadécatriénoïque et ses stéréoisomères, et 4) l'acide arachidonique ou acide 5, 8,11,14-eicosatétraénoïque et ses stéréoisomères.

La vitamine F est essentiellement constituée d'acide linoléique et de ses isomères, l'isomère 9,12 étant présent dans une proportion pouvant varier entre environ 40 et 70 %, la proportion totale d'acides linoléiques (acide linoléique + isomères) représentant environ de 80 à 90 %, le reste étant essentiellement constitué par un mélange d'autres acides gras essentiels.

Selon cette forme de réalisation, l'acide gras essentiel pris seul ou en mélange, est généralement présent en une proportion comprise entre 0,2 et 20 % en poids par rapport au poids d'huile de karité.

Afin d'améliorer la stabilité des compositions, celles-ci peuvent également contenir au moins un agent anti-oxydant tel que par exemple le butylhydroxyanisole (BHA) ou le butylhydroxytoluène (BHT) ou un mélange de ces substances à raison d'environ 0,002 à 0,2 % par rapport au poids total de la composition.

On va maintenant donner à titre d'illustration et sans aucun caractère limitatif plusieurs exemples de compositions cosmétiques aqueuses ou anhydres selon l'invention.

**Exemple 1:**

Crème de soins sous forme d'une 'emulsion huile-dans-l'eau

| | |
|---|---|
| Phase grasse: | |
| - alcool cétylique | 0,5 |
| - Huile de karité | 18 |
| - Perhydrosqualène de synthèse | 4 |
| - Stéarate de glycérol | 2 |
| - Tween·60® (monostéarate de sorbitan à 20 moles d'oxyde d'éthylène) | 1 |
| - Acide stéarique | 1,4 |
| - Triéthanolamine | 0,7 |
| - Carbopol 940® (neutralisé par triéthanolamine) | 0,4 |
| - Vitamine F | 2 |
| - Anti-oxydant (BHA + BHT) | 0,015 |
| - Parfum | 1 |
| - Eau + conservateur (parahydroxybenzoate de méthyle) QSP | 100 % en poids |

**Exemple 2:**

Crème de soins sous forme d'une émulsion eau-dans-l'huile

Phase grasse:
- Cire microcristalline ................................... 1
- Huile de vaseline ...................................... 5
- Huile de germe de maïs ............................... 2
- Huile de karité ........................................ 12
- Esters d'acide gras en C 8-C 18 et d'alcool gras en C 12-C 18 ... 1
- Monoisostéarate de sorbitan ......................... 5
- Gel de montmorillonite modifiée organiquement et d'huile neutre
(triglycérides d'acides caprylique et caprique) .......... 3
- Propylène glycol ...................................... 3
- BHA + BHT ........................................... 0,01
- Eau + conservateur QSP ............................. 100 % en poids

**Exemple 3:**

Crème de soins sous forme d'une émulsion eau-dans-l'huile

Phase grasse:
- myristate d'isopropyle ............................... 8
- Huile de paraffine ..................................... 18
- Huile de karité ........................................ 22
- Ozokérite ............................................. 4
- Lanolate de magnésium ............................... 14,4
- Alcool de lanoline ..................................... 3,6
- BHA + BHT ........................................... 0,01
- Eau + parahydroxybenzoate de méthyle QSP ......... 100 % en poids

**Exemple 4:**

Lait démaquillant

Phase grasse
- Huile de vaseline ...................................... 6
- Palmitate d'isopropyle ................................ 5
- Huile de karité ........................................ 6
- Stéarate de glycérol ................................... 2
- Acide stéarique ........................................ 1,4
- Triéthanolamine ....................................... 0,7
- Copolymère anhydride maléique/méthyl vinyl
éther réticulé (Viscofas X 100.000) .................... 0,6
- Lysine ................................................. 0,5
- Antioxydant (BHA + BHT) ............................ 0,002
- Eau + conservateur QSP ............................. 100 % en poids

**Exemple 5:**

Crème démaquillante

Phase grasse
- Huile de karité ........................................ 15
- Stéarate de glycérol ................................... 2
- Acide stéarique ........................................ 1,4
- Triéthanolamine ....................................... 0,7
- Carbopol 940® (GOODRICH) .......................... 0,3
- Lysine ................................................. 0,5
- BHA + BHT ........................................... 0,015
Eau + Conservateur QSP ............................... 100 % en poids

6

**Exemple 6:**

Crème savon à rincer

Phase grasse
- Huile minérale                                    15
- Huile de karité                                   5
- Stéarate de triéthanolamine                       12
- Propylène glycol                                  10
- Viscofas X 100.000®                               0,4
- Arginine                                          0,35
- BHA + BHT                                         0,005
- Eau + conservateur QSP                            100 % en poids

**Exemple 7:**

Crème teinté sous forme d'un émulsion huile-dans-l'eau

Phase grasse
- Glycérides partiels d'acides gras                 8
- Alcool cétylique                                  0,5
- Décylester d'acide oléique                        8
- Huile de vaseline                                 4
- Huile de karité                                   16
- Ether polyglycolique d'alcool gras saturé         4
- Silicate de magnésium et d'aluminium              0,7
- Poudre de polyéthylène (Polymist B6 ®
de la Société ALLIED CHEMICALS)                     4
- Oxydes de fer                                     2,2
- BHA + BHT                                         0,08
- Eau + conservateur QSP                            100 % en poids

**Exemple 8:**

Fond de teint sous forme d'une émulsion eau-dans-l'huile

Phase grasse
- Huile de paraffine                                10
- Huile de karité                                   10
- Huile de purcellin                                4
- Perhydrosqualène                                  6
- Ozokérite                                         2
- Lanolate de magnésium                             5
- Alcool de lanoline                                3
- Oxydes de fer                                     3
- Dioxyde de titane                                 4
- Poudre de polyéthylène                            10
- Parfum                                            0,4
- BHA + BHT                                         0,01
- Eau + Conservateur QSP                            100 % en poids

7

**Exemple 9:**

Crème solaire

Phase grasse

| | |
|---|---|
| - Huile de vaseline | 34 |
| - Huile de karité | 12 |
| - Cire d'abeilles | 3 |
| - Lanolate de magnésium | 2,4 |
| - Alcool de lanoline | 0,6 |
| - Poudre de polyéthylène | 10 |
| - BHA + BHT | 0,01 |
| - Filtre solaire "EUSOLEX 6300®" (de la Société MERCK) | 3,5 |
| - Parfum | 1 |
| - Eau + conservateur QSP | 100 % en poids |

**Exemple 10:**

Lait corporel

Phase grasse

| | |
|---|---|
| - Huile de karité | 3 |
| - Huile de vaseline | 8 |
| - Stéarate de glycérol | 2 |
| - Tween 60® (monostéarate de sorbitan à 20 moles d'oxyde d'éthylène) | 1 |
| - Acide stéarique | 1,4 |
| - Triéthanolamine | 0,7 |
| - Carbopol 940 (neutralisé par de la triéthanolamine) | 0,2 |
| - BHA + BHT | 0,01 |
| - Parfum | 1 |
| - Eau + conservateur QSP | 100 % en poids |

**Exemple 11:**

Stick de soins pour les lèvres

| Phase grasse | grammes |
|---|---|
| - Cire microcristalline | 10 |
| - Cire de Candellila | 5 |
| - Lanoline | 76,9 |
| - Lanoline liquide | 1,6 |
| - Huile de karité | 12 |
| - Huile minérale épaisse | 8 |
| - Ricinoléate de cétyle | 20 |
| - Vitamine F (acides gras essentiels) | 2 |
| - Antioxydant | 0,1 |

**Exemple 12:**

Rouge à lèvres dont le corps blanc a la composition suivante:

| Phase grasse | grammes |
|---|---|
| - Cire microcristalline | 15 |
| - Polylaurate de vinyle | 10 |
| - Docosanoyloxy - 1 (éthyl-2) hexyloxy-3 propanol | 10 |
| - Huile de ricin | 25 |
| - Alcool cétylique | 2,5 |
| - Triglycérides des acides caprique/caprylique | 3,3 |
| - Huile de karité | 10 |
| - Lanoline acétylée | 5 |
| - Propionate d'arachidyle | 5 |
| - Huile de sésame | 10 |
| - Acétoglycérides | 4 |
| - Antioxydant | 0,2 |

**Exemple 13:**

Baume anhydre

| Phase grasse | grammes |
|---|---|
| - Huile de karité | 60 |
| - Huile de sésame | 20 |
| - Vaseline® | 15 |
| - Lécithine de soja | 4,8 |
| - BHA | 0,1 |
| - BHT | 0,1 |

**Exemple 14:**

Gel démaquillant anhydre

| Phase grasse | grammes |
|---|---|
| - Vaseline® filante | 50 |
| - Palmitate d'isopropyle | 20 |
| - Huile de paraffine | 20 |
| - Huile de karité + antioxydant | 9,8 |
| - (BHA + BHT) | 0,2 |

**Exemple 15:**

Huile pour le visage et le corps

| Phase grasse | grammes |
|---|---|
| - Huile de karité | 2 |
| - Huile de soja | 32 |
| - Huile de tournesol | 31,8 |
| - Huile d'arachide | 32 |
| - BHA + BHT | 0,2 |
| - Vitamine F (acides gras essentiels) | 0,4 |

9

**Exemple 16:**

Huile solaire

| Phase grasse | grammes |
|---|---|
| - Huile de karité | 1 |
| - Huile de colza QSP | 100 |
| - 2-éthyl hexyl paraméthoxy cinnamate vendu sous la dénomination de "PARSOL MEX®" par la Société GIVAUDAN | 2,5 |
| - BHA | 0,025 |
| - BHT | 0,025 |
| - Colorant QS | |
| - Parfum QS | |

## Revendications

1. Composition cosmétique, aqueuse ou anhydre, contenant une phase grasse, caractérisée par le fait que ladite phase grasse est essentiellement constituée par de l'huile de karité, ou par un mélange d'huile de karité et d'au moins une autre huile, une graisse et/ou une cire cosmétique, ladite huile de karité provenant du fractionnement du beurre de karité à l'aide d'un solvant organique.

2. Composition selon la revendication 1, caractérisée par le fait que l'huile de karité présente les caractéristiques suivantes:

| | |
|---|---|
| - Acide palmitique ($C_{16}$) | 3-5 % |
| - Acide stéarique ($C_{18}$) | 22-30 % |
| - Acide oléique ($C_{18:1}$) | 55-65 % |
| - Acide linoléique ($C_{18:2}$) | 0,5-1,5 % |
| - Acide arachidique ($C_{20}$) | 0,5-1,5 % |
| (Ces pourcentages étant exprimés par rapport au total des acides gras) | |
| - Viscosité: | 100-300 mPa.s (cP) |
| - Point de fusion: | 5,1-5,4° C |
| - Cristallisation: | 2,9-3° C |
| - Insaponifiable: | 1,2-1,5 % |

3. Composition selon la revendication 2, caractérisée par le fait que l'huile de karité présente, les rapports d'acides gras suivants:

$$\frac{C_{16} + C_{18}}{C_{18:1}} = 0,518 \pm 0,01$$

$$\frac{C_{16}}{C_{18:1}} = 0,064 \pm 0,003$$

4. Composition selon l'une quelconque des revendications 1 à 3, caractérisée par le fait que l'huile de karité provient du fractionnement du beurre de karité à l'aide d'acétone.

5. Composition selon l'une quelconque des revendications précédentes, caractérisée par le fait que l'huile de karité est présente à une concentration de 1 à 80 % en poids par rapport au poids total de la composition.

6. Composition selon la revendication 1, caractérisée par le fait que ladite phase grasse est présente en une proportion de 4 à 99,9 % en poids par rapport au poids total de la composition.

7. Composition cosmétique selon l'une quelconque des revendications précédentes, caractérisée par le fait qu'elle se présente sous forme d'une émulsion du type eau-dans-l'huile ou huile-dans-l'eau, la phase grasse représentant de 4 à 60 % en poids, la phase eau de 30 à 90 % et l'agent émulsionnant de 1 à 20 % mais de préférence de 2 à 12 % en poids.

8. Composition selon la revendication 7, caractérisée par le fait que la phase grasse contient de préférence de 2 à 30 % en poids d'huile de karité par rapport au poids total de la composition.

9. Composition selon l'une quelconque des revendications précédentes, caractérisée par le fait qu'elle contient au moins un acide gras essentiel ou un mélange de ces acides, notamment de la vitamine F, sous forme acide libre ou estérifiée.

10. Composition selon la revendication 9, caractérisée par le fait que l'acide gras pris seul ou en mélange est présent à une concentration comprise entre 0,2 et 20 % en poids par rapport au poids d'huile de karité.

11. Composition selon l'une quelconque des revendications précédentes, caractérisée par le fait qu'elle contient en outre un agent anti-oxydant tel que le butylhydroxyanisole ou le butylhydroxytoluène ou un mélange de ses substances à raison d'environ 0,002 à 0,2 % par rapport au poids total de ladite composition.

12. Composition selon l'une quelconque des revendications précédentes, caractérisée par le fait qu'elle contient en outre divers ingrédients tels que des agents colorants, des parfums, des conservateurs, des filtres UV, des pigments, des agents nacrants et des charges minérales ou organiques.

**0 145 607**

## Patentansprüche

1. Wäßriges oder wasserfreies kosmetisches Mittel, das eine Fettphase enthält, dadurch gekennzeichnet, daß die Fettphase im wesentlichen aus Karitéöl oder einer Mischung aus Karitéöl und mindestens einem weiteren Öl, einem Fett und/oder einem kosmetischen Wachs besteht, wobei das Karitéöl aus der Fraktionierung von Karitébutter mit Hilfe eines organischen Lösungsmittels stammt.

2. Mittel nach Anspruch 1, dadurch gekennzeichnet, daß das Karitéöl folgende Charakteristika besitzt:

| | |
|---|---|
| Palmitinsäure ($C_{16}$) | 3-5 % |
| Stearinsäure ($C_{18}$) | 22-30 % |
| Oleinsäure ($C_{18:1}$) | 55-65 % |
| Linolsäure ($C_{18:2}$) | 0,5-1,5 % |
| Arachinsäure ($C_{20}$) | 0,5-1,5 % |
| (Diese Prozentangaben sind bezogen auf das Gesamtgewicht der Fettsäuren) | |
| Viskosität: | 100-300 mPa.s (cP) |
| Schmelzpunkt: | 5,1-5,4°C |
| Kristallisation: | 2,9-3°C |
| Unverseifbarer Anteil: | 1,2-1,5 % |

3. Mittel nach Anspruch 2, dadurch gekennzeichnet, daß das Karitéöl die folgenden Fettsäureanteile aufweist.

$$\frac{C_{16} + C_{18}}{C_{18:1}} = 0,518 \pm 0,01$$

$$\frac{C_{16}}{C_{18:1}} = 0,064 \pm 0,003$$

4. Mittel nach einem der Ansprüche 1 bis 3, dadurch gekennzeichnet, daß das Karitéöl aus der Fraktionierung von Karitébutter mit Hilfe von Aceton stammt.

5. Mittel nach einem der vorhergehenden Ansprüche, dadurch gekennzeichnet, daß das Karitéöl in einer Konzentration von 1 bis 80 Gew.-%, bezogen auf das Gesamtgewicht des Mittels, vorhanden ist.

6. Mittel nach Anspruch 1, dadurch gekennzeichnet, daß die Fettphase in einem Anteil von 4 bis 99,9 Gew.-%, bezogen auf das Gesamtgewicht des Mittels, vorhanden ist.

7. Kosmetisches Mittel nach einem der vorhergehenden Ansprüche, dadurch gekennzeichnet, daß es als Wasser-in-Öl oder Öl-in-Wasser-Emulsion vorliegt, wobei die Fettphase 4 bis 60 Gew.-%, die wäßrige Phase 3 bis 90 Gew.-% und der Emulgator 1 bis 20 Gew.-%, vorzugsweise 2 bis 12 Gew.-%, ausmachen.

8. Mittel nach Anspruch 7, dadurch gekennzeichnet, daß die Fettphase vorzugsweise 2 bis 30 Gew.-% Karitéöl enthält, bezogen auf das Gesamtgewicht des Mittels.

9. Mittel nach einem der vorhergehenden Ansprüche, dadurch gekennzeichnet, daß es mindestens eine essentielle Fettsäure oder eine Mischung dieser Säuren, insbesondere Vitamin F, als freie Säure oder als Ester enthält.

10. Mittel nach Anspruch 9, dadurch gekennzeichnet, daß die alleine oder in Mischung vorliegende Fettsäure in einer Konzentration von 0,2 bis 20 Gew.-%, bezogen auf das Gewicht des Karitéöls, vorhanden ist.

11. Mittel nach einem der vorhergehenden Ansprüche, dadurch gekennzeichnet, daß es außerdem ein Antioxidans, wie Butylhydroxyanisol oder Butylhydroxytoluol, oder eine Mischung dieser Substanzen in einem Anteil von 0,002 bis 0,2 %, bezogen auf das Gesamtgewicht des Mittels, enthält.

12. Mittel nach einem der vorhergehenden Ansprüche, dadurch gekennzeichnet, daß es außerdem andere Bestandteile, wie Farbstoffe, Parfüms, Konservierungsmittel, UV-Filter, Pigmente, perlmuttschimmerverleihende Mittel und mineralische oder organische Füllstoffe, enthält.

## Claims

1. Aqueous or anhydrous cosmetic composition containing a fatty phase, characterized in that the said fatty phase essentially consists of shea oil, or of a mixture of shea oil and at least one other oil, a fat and/or a cosmetic wax, the said shea oil originating from the fractionation of shea butter using an organic solvent.

2. Composition according to Claim 1, characterized in that the shea oil has the following characteristics:

| | |
|---|---|
| Palmitic acid (C$_{16}$) | 3-5 % |
| Stearic acid (C$_{18}$) | 22-30 % |
| Oleic acid (C$_{18:1}$) | 55-65 % |
| Linoleic acid (C$_{18:2}$) | 0.5-1.5 % |
| Arachidic acid (C$_{20}$) | 0.5-1.5 % |
| (These percentages are expressed relative to the total of the fatty acids) | |
| Viscosity: | 100-300 mpa.s (cP) |
| Melting point: | 5.1-5.4° C |
| Crystallization: | 2.9-3° C |
| Unsaponifiables: | 1.2-1.5 % |

3. Composition according to Claim 2, characterized in that the shea oil has the following fatty acid ratios:

$$\frac{C_{16} + C_{18}}{C_{18:1}} = 0.518 \pm 0.01$$

$$\frac{C_{18}}{C_{18:1}} = 0.064 \pm 0.003$$

4. Composition according to any one of Claims 1 to 3, characterized in that the shea oil originates from the fractionation of shea butter using acetone.

5. Composition according to any one of the preceding claims, characterized in that the shea oil is present in a concentration from 1 to 80 % by weight relative to the total weight of the composition.

6. Composition according to Claim 1, characterized in that the said fatty phase is present in a proportion from 4 to 99.9 % by weight relative to the total weight of the composition.

7. Cosmetic composition according to any one of the preceding claims, characterized in that it is in the form of an emulsion of the water-in-oil or oil-in-water type, the fatty phase representing from 4 to 60 % by weight, the water phase from 30 to 90 % and the emulsifying agent from 1 to 20 %, but preferably from 2 to 12 %, by weight.

8. Composition according to Claim 7, characterized in that the fatty phase preferably contains from 2 to 30 % by weight of shea oil relative to the total weight of the composition.

9. Composition according to any one of the preceding claims, characterized in that it contains at least one essential fatty acid or a mixture of these acids, especially of vitamin F, in the free acid or the esterified form.

10. Composition according to Claim 9, characterized in that the fatty acid taken alone or as a mixture is present in a concentration of between 0.2 and 20 % by weight relative to the weight of the shea oil.

11. Composition according to any one of the preceding claims, characterized in that it additionally contains an antioxidant such as butylated hydroxyanisole or butylated hydroxytoluene or a mixture of these substances in a proportion of approximately 0.002 to 0.2 % relative to the total weight of the said composition.

12. Composition according to any one of the preceding claims, characterized in that it additionally contains other ingredients such as colouring agents, perfumes, preservatives, UV filters, pigments, agents giving a pearly lustre and inorganic or organic fillers.